# EUROPEAN PATENT SPECIFICATION

(11) **EP 2 058 385 B1**
(45) Date of publication and mention of the grant of the patent: **07.11.2018**
(21) Application number: 08425633.8
(22) Date of filing: 26.09.2008
(51) Int. Cl.: C12G 1/02

(54) **Fermentation apparatus**
Fermentationsvorrichtung
Appareil de fermentation

(30) Priority: 12.11.2007 EP 07425706
(43) Date of publication of application: 13.05.2009
(73) Proprietor: Noform S.r.l., 30020 Meolo (VE) (IT)
(72) Inventor: Crosato, Remo, 31048 S. Biagio di Callalta Treviso (IT); Crosato, Stefano, 31048 S. Biagio di Callalta Treviso (IT)
(74) Representative: Citron, Massimiliano

(56) References cited:
- EP-A- 0 530 820
- EP-A- 1 314 778
- EP-A- 1 616 937
- WO-A-2005/023977
- WO-A-2006/087601
- US-A- 4 021 579

## Description

The invention relates to a fermentation apparatus.

Although the invention is useful for the fermentation of any vegetable product in the form of a crushed material, the description that follows will refer as an example to winemaking, where the invention has proved particularly effective.

Winemaking is carried out with the help of special tanks, where the must is introduced to ferment. The fermentation process generates large amounts of gaseous products, especially CO2, participating actively to the success of a good wine. The gases are released from the must and push the marc and every solid part upward where they compact and form a solid layer, called "cap".

The winemaking methods exploit the fermentation gases, as in WO 98/45403, to mix the cap and prevent solidification thereof. In combination or not, other methods consider to adjust the pressure inside the winemaking tank through safety or degassing valves, both mechanical or membrane valves.

An example of pressure adjustment is described in WO 2006/087601. Here a particular type of degassing valve is used, of the membrane type, in which fluid is pumped to tighten it and lock the vent thereof.

Recently, the applicant has surprisingly discovered how much the pressure inside the winemaking tank is very important, especially during the degassing. Too high a pressure in the tank has immediate repercussions on the quality of wine. The degassing, impulsive or not, induce the must to surface on the cap and the release of gases from within the grape berries. If the pressure is too high, the berries break and a lot of dregs (mush suspended in the must) is produced. It has been discovered that each grape has its optimum fermentation pressure, which must be regulated with a maximum tolerance of one hundredth of bar (e.g. values as precise as 0.29 bar or 0.31 bar).

The membranes in WO 2006/087601 are made of rubber or similar, and suffer greatly from many factors such as their inertia, whereby they regulate very approximately their venting, or the temperature. A pressure maintained at 0.3 bar during the day can move also to 0.2 bar at night, when the cellar is colder. Moreover, the more the internal pressure of the tank is approaching the threshold pressure, the more sensitivity and precision is lost in the valve, because the membranes are beginning to open. Swinging values with errors of 0.1 bar in WO 2006/087601, which is about 25% of the usual maximum working pressure, are not adequate for a controlled-pressure fermentation. Not only can the must be denatured, but also could the proper fermentation process of grapes be altered through wrong pressures. The same goes for mechanical valves with spring pressure adjustment and calibration.

EP 1 314 778 discloses a wine-making apparatus provided with a fermenter and an external tank. The tank can be set in communication with the upper part of the fermenter to transfer thereto must taken from the tank.

This and known winemaking apparatuses can be improved, and such is the object of the present invention, which wants to present an apparatus for the fermentation of a vegetable product in the form of crushed material, preferentially must, which allows to establish inside a fermentation (e.g. vinification) tank a pressure of the gases produced by fermentation with very precise value.

Another object is to enable the setting of programmed pressure trends inside the (e.g. vinification) tank.

This object is achieved with an apparatus for the fermentation of a vegetable product in the form of crushed material, preferentially must, comprising the features of claim 1.

With the invention the pressure inside either, or both, tanks can be adjusted very finely (with error about the hundredth of bar). In the standard pressure range used (0,15-0,5 bar) an enologist or generally a user can customize the value and/or trend of pressure over time, for example in a pump-over cycle and/or on different cycles in sequence.

All this provides the wine specialist enormous potential for winemaking. As mentioned above the pressure in the vinification tank affects a lot the quality and type of wine produced. Variables such as the type of grape, vintage, temperature and the degree or stage of fermentation combine with the pressure variable in a single recipe to give the final wine.

In addition, the apparatus of the invention better adapts to receive more types of grapes, because it can process them creating a fermentation environment precisely pressure-controlled. For each grape specific parameters of pressure are needed, although very different from grape to grape; with the invention, the winemaking apparatus is able to offer an optimal and adaptable environment for vinification.

Thus, it is preferred that the adjustment means are adapted to adjust the gaseous products pressure with an error less than or equal to 0.01 bar.

To achieve such great precision, preferably the adjustment means comprise valve means, a processing unit, a pressure sensor for detecting the inner pressure of the tank to be stabilized, the unit being adapted to read the data outputted by the sensor and adjust the third valve means so as to maintain the pressure on a programmed value. In practice, a feedback control is carried out.

As valve means one may use:
- a single valve, mounted between the first tank and the outside, or
- a single valve, mounted on the first piping system between the first valve means and the second tank;
- two valves, one rough and one more precise in parallel, placed on the first tank or the first piping system.

Preferably the valves are of the open/close type, so that the processing unit can constantly open and close the additional valve(s) to vent little excess gas at a time with respect to the programmed pressure.

Much depends on the volume of gas to degas. For a very large tank, there may be a sole valve and also of not refined mechanics, because the inaccuracy about its vent flow becomes negligible compared to a much larger volume of gas. Conversely, for smaller tanks there is needed a valve with precise and controlled vent, so for example in parallel to a safety valve, used for violent instantaneous degassing, a small electromechanical open/close valve may be placed. The whole meets the specific demands of accuracy.

However, a slower degassing can always happen with a single pressure finely-regulating valve.

To make clear the benefits of the invention, a preferred apparatus will now be described according to the invention with reference to the drawings, where
Figures 1-4 show a winemaking apparatus according to the invention in various steps of operation;
Fig. 5 shows another preferred form of the winemaking apparatus according to the invention, denoted by 50.

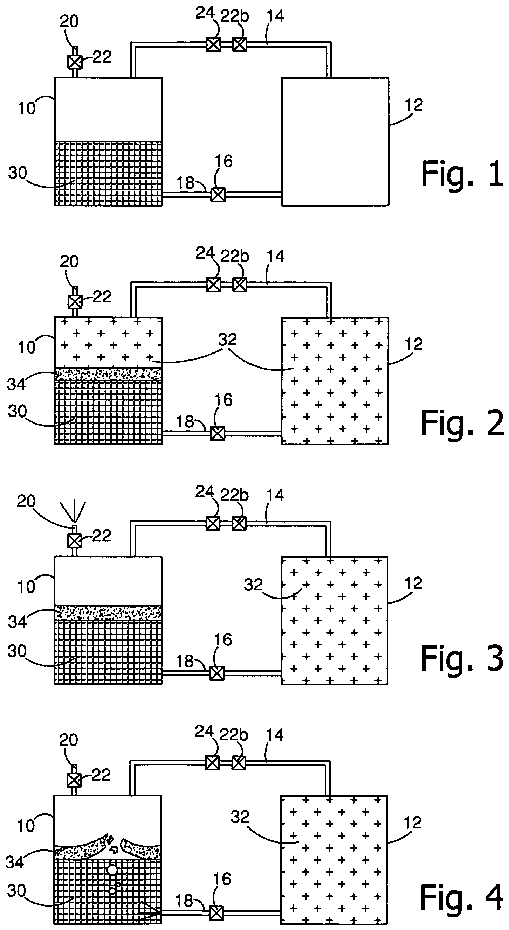

A tank 10 (Fig. 1) is filled by known means and ways with must 30. A conduit 14 puts in communication in a controllable manner, through a valve 24, the upper part of the tank 10 with the upper part of a second tank 12. A second conduit 18 puts into communication in a controllable manner, through a valve 16, the bottom of the tank 10 with the bottom of the tank 12. The tank 10 has an upper vent 20 controllable by pressure adjusting means 22. The valve 16 is initially closed to block a reflux of the must 30.

After a certain time (Fig. 2) the must 30 generates fermenting a gas or gaseous products 32 (mainly CO2) and creates a solid cap 34. The valve 24 is open and spontaneously the gas 32 invades the tank 12. The pressure adjusting means 22 are regulated to prevent the escape of the gas 32 from the tank 10.

After a predetermined time, the tanks 10, 12 are isolated by closing the valve 24. The gas 32 gets trapped in pressure in the tank 12, while the other is depressurized (or degassed) through the conduit 20 by adjusting the pressure adjusting means 22 so as to open the conduit 20 (Fig. 3).

Finally, (Fig. 4), the valve 16 opens and the gas 32 flows spontaneously in the must 30. While going up it will determine the soft rupture of the cap 34 and its leaching, thereby extracting the natural flavorings and colorings therefrom.

The pressure adjusting means 22 are controlled by an electronic unit (not shown), e.g. a PLC, which manages all the steps of winemaking. This unit may implement programming user interfaces and driving stages for the pressure adjusting means 22. Through control of the means 22, the unit can accurately maintain any particular planned pressure inside the tank 10. To this aim, the unit is interfaced with a pressure sensor (not shown) which measures the gas pressure in the tank 10.

As a pressure adjusting means one can use valve means, for example a single valve, mounted between the first tank 10 ant the external environment or two, one rough and one more precise in parallel. Preferably the valves are of the open/close type, so that the processing unit can constantly open and close the additional valve(s) to vent a little gas at a time.

The pressure inside the gas accumulation tank 12 is very important. In fact, too high a pressure in this tank has a huge influence on wine quality. The release, impulsive or not, of the gas 32 stored under pressure from the tank 12 to tank 10 (and below the marc cap), induces a more or less violent break of the marc cap, the surfacing of must on the cap and the release of gas from the inside of grape berries. If the pressure is too high, the berries break and a lot of dregs is produced (mush suspended in the must).

It has been discovered that each and every grape and marc cap (in connection both to vintage, and to the harvest time of grapes and even the same manner in which the grapes are collected, e.g. by mechanical or manual harvesting) has its optimum pressure for the pump-over gas, which must be regulated with a maximum tolerance of one hundredth of bar (e.g. as precise a value as 0.29 bar or 0.31 bar).

It may be understood how the invention ensures even in this case the optimal fermentation environment for the must.

In Figures 1-4 is shown another valve 22b, optional or in replacement to the valve 22, placed on the conduit 14 with the vent directed to the outside. In particular, when the valve 22b is present, the apparatus allows more control to be performed over winemaking. Consider, for example, the case wherein the valve 22 is absent (reduced number of components).

The gas storage in the tank 12, with the valve 24 open, occurs as stated above, but the valve 22b allows in this step to control the pressure of gas 32 in the tank 10, i.e. there is the control of the pressure which the must 30 and the cap 34 experience during fermentation.

With the valve 24 closed the gas 32 gets trapped in the tank 12, as already mentioned, but the valve 22b now allows when necessary to control the pressure in the tank 12. This allows to adjust the pressure of the gas 32 which knocks the must 30 and the cap 34 in the next step of pumping-over.

It is clear how in this case the invention with only two vent valves ensures absolute control of all the crucial pressures in the winemaking apparatus.

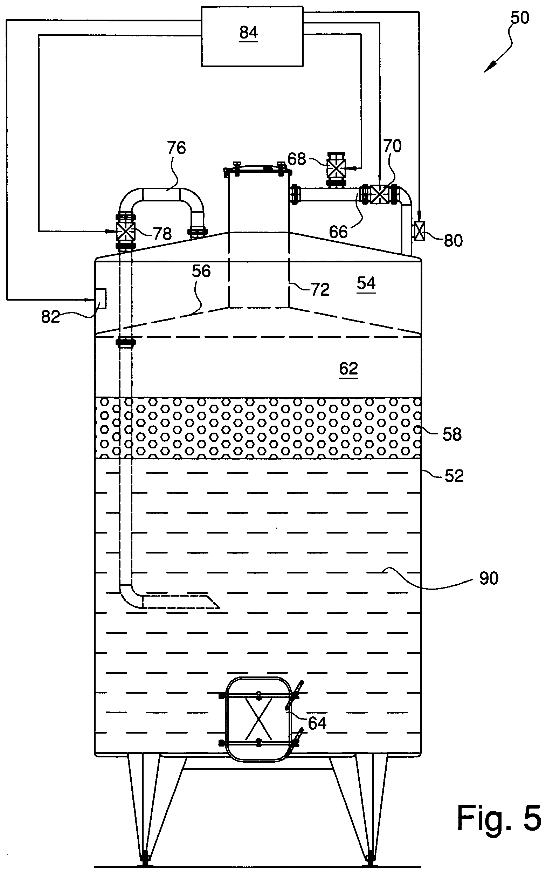

For a more compact version and with two valves see fig. 5. A winemaking apparatus 50 for some must 90 consists of a cylindrical casing 52 internally divided by a conical-cap separating wall 56 in two stacked sub-tanks or volumes 54, 62, upper and lower respectively.

The volume 62 communicates with the outside through a base hatch 64 and a vertical hatch 72, around which the tank 54 develops.

The upper tank 54 is selectively connectable to the hatch 72 via a conduit 66 and a valve 70. On the same conduit 66 there is a second valve 68 with the function of making communicating the hatch 72 (and thus the tank 62) with the outside world for the degassing (when its closing door 73 is closed). On the conduit 66, between the valve 70 and the tank 54, is mounted another valve 80 of the electromechanical open/close type, smaller than the valve 68, and therefore more sensitive and with limited flow. Its goal is to finely adjust the pressure inside the tanks 62, 54.

The tank 54 is also selectively connectable to the tank 62 via a conduit 76 and a valve 78.

The valves 70, 68, 78, 80 are controlled by an electronic unit 84, for example a PLC, which manages all the steps of winemaking. This unit 84 may implement timers, programming user interfaces and driving stages for the valves 68, 70, 78, 80 (see arrows in Fig. 5).

Through the control of the valve 80, specially designed to release a small amount of gas at a time to the outside (i.e. it has a very small controllable flow so as to regulate the hundredth of bar in the tank 62 and/or 54), the unit 84 can maintain precisely any particular planned pressure inside the tank 62 and/or 54. For this aim, the unit 84 is interfaced with a pressure sensor 82, which measures the gas pressure in the tank 54.

The operation of the winemaking apparatus 50 is the same as described for Figures 1-4, being enough to keep in mind the following correspondence:
tank 10 ↔ tank 62
tank 12 ↔ tank 54
valve 22 ↔ valve 68
valve 24 ↔ valve 70
valve 16 ↔ valve 78
conduit 14 ↔ conduit 66
conduit 18 ↔ conduit 76
valve 22b ↔ valve 80.

The overall operation will be repeated succinctly for the main steps.

Basically, the proper driving of the valves 70, 68, 78 allows to accumulate in the tank 54 the gaseous products of fermentation in order then to discharge them into the tank 62 to break a marc cap 58.
(i) The tank 62 is filled with must 90.
(ii) After some time the fermenting must 90 generates some gas and a solid cap 58. The valve 70 is open, the valve 68 and the valve 78 are closed. Therefore spontaneously the gas invades tank 54. The valve 80 will be adjusted, for example with cycles having proper duty-cycle of short opening alternated to closure, so as to maintain the two tanks 54, 62 at a predetermined pressure, defined by the user by programming the unit 84. Such pressure can be either constant or follow a programmed reference trend, and corresponds to the pressure which the must 90 and the cap 58 undergo.
(iii) After a scheduled time, the valve 70 is closed. The gas remains trapped in pressure in the tank 54, while the other tank 62 is depressurized (or degassed) by opening the valve 68, which is designed to withstand even a violent outflow. Now with the valve 80 the pressure in the tank 54 can be adjusted to a value even different from the previous one (by also introducing, or not, into the tank 54 compressed air or other process-compatible gases).
(iv) the valve 78 is opened and the gas flows spontaneously into the must 30 and while going up it interacts with the cap 58, breaking it. Because the gas pressure was adjusted to a precise value, which is the optimal one for the processed grapes, the interaction with the must and the cap is the best possible.

It is clear that every phase (i-iv) can be advantageously automated and/or programmed by the unit 84. This allows to set cycles, periodic or not, of breaking the cap 58, and to test various methods of degassing the tank 62. In particular the best value, and/or that trend, for the pressure in the tanks 54, 62 (values and trends also being independent between each other) can be found, which gives optimal results for the wine. The same applies to the apparatus of fig. 1.

## Claims

1. Apparatus (50) for the fermentation of a vegetable product in the form of crushed material, preferentially must, comprising
- a first tank (62) for containing the crushed material (90) and a second collection tank (54) for the gaseous products generated from the crushed material's fermentation,
- a first piping system (66) adapted to let communicate a part of the first tank, where the gaseous products are gathered, with the second tank,
- a second piping system (76) adapted to let communicate the second and the first tank, the system having an outlet in the first tank where, in use, the liquid of the crushed material is present,
- first and second valve means (70, 78) associated respectively to the first and second piping system to make the two tanks selectively communicating conditionally to the open/closed status of said means;
**characterized by**
comprising adjustment means (80, 82, 84) adapted to finely adjust the gaseous products' pressure at one or more values programmed by a user inside the first and second tank when they are communicating and/or inside the second tank when it is isolated,
wherein the adjustment means are adapted to adjust the gaseous products' pressure with an error less than or equal to 0.01 bar.

2. Apparatus according to claim 1, wherein the adjustment means comprise
- third valve means (80),
- a processing unit (84),
- a pressure sensor (82) for detecting the inner pressure of the tank to be stabilized,
the unit being adapted to read the data outputted by the sensor and adjust the third valve means so as to maintain the pressure on a programmed value.

3. Apparatus according to claim 2, wherein the third valve means comprise a valve (80) mounted for venting the tank to be stabilized towards the outside.

4. Apparatus according to claim 3, wherein said valve is mounted on the first piping system (66) between the first valve means (70) and the second tank (54).

5. Apparatus according to one of claims 2 to 4, wherein the third valve means comprise a valve (68) mounted for venting the first tank towards the outside and destined to instantly degas the first tank towards the outside, and a second valve (80) connected in parallel and adapted to finely adjust the pressure of the tank to be stabilized.

6. Apparatus according to one of claims 2 to 5, wherein the third valve means are valves of the open/close type.

7. Apparatus according to one of claims 2 to 6, wherein the pressure sensor is mounted to monitor the pressure in the second tank.

8. Apparatus according to one of claims 2 to 7, wherein the processing unit is provided with a memory and is programmed and/or programmable to adjust, through driving of the third valve means, the pressure in the first tank so that it follows a programmed reference trend stored in the memory.

9. Apparatus according to any one of the preceding claims, comprising an outer casing (52) divided internally by at least one separating wall (56) in two sub-volumes which form the first and the second tank.

10. Apparatus according to claim 9, wherein said first and second tank are arranged vertically one over the other inside the casing.

11. Apparatus according to claim 9, wherein said first and second tank are two physically independent tanks (10, 12) connected together through a first and second piping system (14, 18).

## Patentansprüche

1. Vorrichtung (50) zur Fermentation eines pflanzlichen Produktes in Form eines Kelterungsproduktes, vorzugsweise Most, umfassend:
- einen ersten Lagertank (62) zum Enthalten des Kelterungsproduktes (90) und einen zweiten Vorlagetank (54) für die aus der Fermentation des Kelterungsproduktes erzeugten gasförmigen Produkte,
- ein erstes Leitungssystem (66), das dazu geeignet ist, ein erstes Teil des zweiten Lagertanks, wo sich die gasförmigen Produkte ansammeln, mit dem zweiten Lagertank miteinander in Kommunikation zu stellen,
- ein zweites Leitungssystem (76), das dazu geeignet ist, den ersten und den zweiten Lagertank miteinander in Kommunikation zu stellen, wobei das System eine Mündung in den ersten Lagertank hat, in der es im Einsatz die flüssige Masse des Kelterungsproduktes gibt,
- die ersten und zweiten Ventilmittel (70, 78), die jeweils dem ersten und zweiten Leitungssystem zugeordnet sind, um die zwei Lagertanke selektiv miteinander in Kommunikation zu stellen, was vom offenen/geschlossenen Zustand der Mittel abhängt;
**dadurch gegenzeichnet, dass**
sie Einstellmittel (80, 82, 84) umfassen, die dazu geeignet sind, den Druck der gasförmigen Produkte bis zu einem oder mehreren Werten fein einzustellen, welche innerhalb des ersten und zweiten Lagertanks, wenn die Lagertanke miteinander kommunizieren, und/oder innerhalb des ersten und zweiten Lagertanks, wenn er isoliert ist, von einem Benutzer programmiert werden,
wobei die Einstellmittel dazu geeignet sind, den Druck der gasförmigen Produkte mit einem Fehler einzustellen, der kleiner oder gleich 0,01 bar ist.

2. Vorrichtung nach Anspruch 1, in der die Einstellmittel
- dritte Ventilmittel (80),
- eine Verarbeitungseinheit (84),
- einen Drucksensor (82) zum Erfassen des Drucks innerhalb des zu stabilisierenden Lagertanks,
umfassen, wobei die Einheit dazu geeignet ist, die vom Sensor ausgegebenen Daten abzulesen und die dritten Ventilmittel so einzustellen, dass der Druck auf einem programmierten Wert bleibt.

3. Vorrichtung nach Anspruch 2, in der die dritten Ventilmittel ein Ventil (80) umfassen, das zum Luftablass des zu stabilisierenden Lagertanks montiert ist, was unter Druck zur äußeren Umgebung geschieht.

4. Vorrichtung nach Anspruch 3, in der das Ventil am ersten Leitungssystem (66) zwischen den ersten Ventilmitteln (70) und dem zweiten Lagertank (54) montiert ist.

5. Vorrichtung nach einem der Ansprüche 2 bis 4, in der die dritten Ventilmittel ein Ventil (68), das zum Luftablass des ersten Lagertanks zur äußeren Umgebung montiert ist und das erste Lagertank instantan nach außen entgasen soll, und ein zweites Ventil (80) umfassen, das parallel verbunden ist und dazu geeignet ist, den Druck des zu stabilisierenden Lagertanks einzustellen.

6. Vorrichtung nach einem der Ansprüche 2 bis 5, in der die dritten Ventilmittel Ventile sind, die vom Typ sind, der geöffnet und wiedergeschlossen werden kann.

7. Vorrichtung nach einem der Ansprüche 2 bis 6, in der der Drucksensor montiert ist, um den Druck des zweiten Lagertanks zu überwachen.

8. Vorrichtung nach einem der Ansprüche 2 bis 7, in der die Verarbeitungseinheit mit einem Speicher versehen ist und so programmiert und/oder programmierbar ist, um durch Steuerung der dritten Ventilmittel den Druck im ersten Lagertank einzustellen, bis er einem im Speicher abgelegten programmierten Referenzverlauf folgt.

9. Vorrichtung nach einem der vorhergehenden Ansprüche, die eine Außenhülle (52) umfasst, die innenseitig durch mindestens eine Trennwand (56) in zwei Untervolumen unterteilt ist, die der erste und zweite Lagertank sind.

10. Vorrichtung nach Anspruch 9, in der das erste und zweite Lagertank innerhalb der Hülle vertikal aufeinander angeordnet sind.

11. Vorrichtung nach Anspruch 9, in der das erste und zweite Lagertank zwei Lagertanke (10, 12) sind, die physisch unabhängig voneinander und über ein erstes und zweites Leitungssystem (14, 18) miteinander verbunden sind.

## Revendications

1. Appareil (50) pour la fermentation d'un produit végétal en forme de matière écrasée, de préférence moût, comprenant :
- un premier réservoir (62) pour contenir la matière écrasée (90) et un second réservoir de collection (54) pour les produits gazeux générées par la fermentation de la matière écrasée,
- un premier système de conduites (66) apte à mettre en communication une partie du premier réservoir, dans laquelle les produits gazeux s'amassent, avec le second réservoir,
- un second système de conduites (76) apte à mettre en communication le second et le premier réservoir, dans lequel le système a un débouché dans le premier réservoir, dans lequel, en service, se trouve la masse liquide de la matière écrasée,
- les premiers et seconds moyens à vanne (70, 78) associés respectivement au premier et second système de conduites pour mettre en communication entre eux les deux réservoirs en fonction de l'état ouvert/fermé desdits moyens ;
**caractérisé en ce**
**qu'**il comprend des moyens de réglage (80, 82, 84) aptes à régler finement la pression des produits gazeux à une ou plusieurs valeurs programmées par un utilisateur à l'intérieur du premier et second réservoir, lorsqu'ils sont en communication entre eux, et/ou à l'intérieur du second réservoir, lorsqu'il est isolé,
dans lequel les moyens de réglage sont aptes a régler la pression des produits gazeux avec une erreur inférieure ou égale à 0,01 bar.

2. Appareil selon la revendication 1, dans lequel les moyens de réglage comprennent
- des troisièmes moyens à vanne (80),
- une unité de traitement (84),
- un capteur de pression (82) pour détecter la pression interne du réservoir à stabiliser,
l'unité étant apte à lire les données émises par le capteur et régler les troisièmes moyens à vanne de manière à maintenir la pression à une valeur programmée.

3. Appareil selon la revendication 2, dans lequel les troisièmes moyens à vanne comprennent une vanne (80) montée pour évacuer les gaz du réservoir à stabiliser en pression vers l'ambiance externe.

4. Appareil selon la revendication 3, dans lequel ladite vanne est montée sur le premier système de conduites (66) entre les premiers moyens à vanne (70) et le second réservoir (54).

5. Appareil selon l'une des revendications 2 à 4, dans lequel les troisièmes moyens à vanne comprennent une vanne (68) montée pour évacuer les gaz du premier réservoir vers l'ambiance externe et destinée à dégaser d'une manière instantanée le premier réservoir vers l'extérieur, et une seconde vanne (80) reliée en parallèle et apte a régler finement la pression du réservoir à stabiliser.

6. Appareil selon l'une des revendications 2 à 5, dans lequel les troisièmes moyens à vanne sont vannes du type qui peut être ouvert et fermé à nouveau.

7. Appareil selon l'une des revendications 2 à 6, dans lequel le capteur de pression est monté pour suivre la pression dans le second réservoir.

8. Appareil selon l'une des revendications 2 à 7, dans lequel l'unité de traitement est munie d'une mémoire et est programmée et/ou peut être programmée pour régler, à travers le pilotage des troisièmes moyens à vanne, la pression dans le premier réservoir, pour qu'elle suive un cours de référence programmé, mémorisé dans la mémoire.

9. Appareil selon l'une quelconque des revendications précédentes, comprenant une coque extérieure (52) subdivisée intérieurement par au moins une paroi de séparation (56) en deux sous-volumes qui constituent lesdits premier et second réservoir.

10. Appareil selon la revendication 9, dans lequel lesdits premier et second réservoir sont disposés verticalement l'un sur l'autre à l'intérieur de la coque.

11. Appareil selon la revendication 9, dans lequel ledit premier et second réservoir sont deux réservoirs (10, 12) physiquement indépendants et reliés entre eux à travers un premier et un second système de conduites (14, 18).
